# EUROPEAN PATENT SPECIFICATION

(11) **EP 3 442 941 B1**
(45) Date of publication and mention of the grant of the patent: **01.12.2021**
(21) Application number: 17782009.9
(22) Date of filing: 24.03.2017
(51) Int. Cl.: C07C 273/18, C07C 275/68

(54) **PROCESS FOR PREPARATION OF CARMUSTINE**
VERFAHREN ZUR HERSTELLUNG VON CARMUSTIN
PROCÉDÉ DE PRÉPARATION DE LA CARMUSTINE

(30) Priority: 16.04.2016 IN 201621013299
(43) Date of publication of application: 20.02.2019
(73) Proprietor: Intas Pharmaceuticals Limited, Ahmedabad - 380054, Gujrat (IN)
(72) Inventor: KAPKOTI, Gobind Singh, Ahmedabad Gujarat 382210 (IN); KUMAR, Nirmal, Ahmedabad Gujarat 382210 (IN); LOURDUSAMY, Mettilda, Québec, QC G1N 4G2 (CA); RADU, Ioan-Iosif, Quebec, QC G1N 4C2 (CA); SHAH, Aakash Maheshkumar, Ahmedabad Gujarat 382210 (IN); MAKWANA, Hitesh Manubhai, Ahmedabad Gujarat 382210 (IN); SHAIKH, Mohamed Zuber Abdulhaq, Ahmedabad Gujarat 382210 (IN); VAGHASIYA, Yogesh Naranbhai, Ahmedabad Gujarat 382210 (IN)
(74) Representative: Accord Healthcare, S.L.U.
(86) International application number: PCT/IB2017/051706
(87) International publication number: WO 2017/178910

(56) References cited:
- EP-A1- 0 902 015
- US-A- 4 028 410
- FRANCA BIGI ET AL: "Selected syntheses of ureas through phosgene substitutes", GREEN CHEMISTRY, vol. 2, no. 4, 2000, pages 140-148, XP055087859, ISSN: 1463-9262, DOI: 10.1039/b002127j
- TAKEDA KAZUYOSHI ET AL: "Studies on activating methods of functional groups. Part X. Convenient methods for syntheses of active carbamates, ureas and nitrosoureas using N,N'-disuccinimido carbonate (DSC)", TETRAHEDRON LET, ELSEVIER, AMSTERDAM, NL, vol. 24, no. 42, 1983, pages 4569-4572, XP009098593, ISSN: 0040-4039
- ANGELICA ION ET AL.: "Synthesis of symmetrical or asymmetrical urea compounds from CO2 via base catalysis", GREEN CHEMISTRY, vol. 9, 8 November 2006 (2006-11-08), pages 159-161, XP002793572, DOI: 10.1039/b612403h
- JP KAMLESH ET AL.: 'Unprecedented ''In Water'' Imidazole Carbonylation: Paradigm Shift for Preparation of Urea and Carbamate' ORG. LETT. vol. 14, no. 11, 18 May 2012, XP055129354
- MIRKO DIKSIC ET AL.: 'Pharmacokinetics of Positron-labeled 1,3-Bis(2-chloroethyl)nitrosourea in Human Brain Tumors Using Positron Emission Tomography' CANCER RESEARCH vol. 44, July 1984, pages 3120 - 3124, XP055420063

## Description

### FIELD OF THE INVENTION

The present invention relates to an improved process for preparation of carmustine (I).

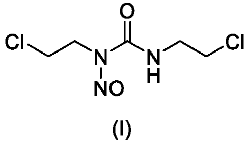

The present invention also relates to preparation of 1,3-bis(2-chloroethyl)urea (II) 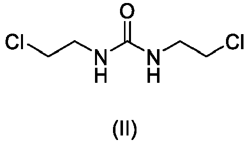 an intermediate used in preparation of carmustine.

### BACKGROUND OF THE INVENTION

"Carmustine" [154-93-8] is a medication used mainly for chemotherapy and sometimes for immunosuppression before transplant. It is a nitrogen mustard β-chloro-nitrosourea compound used as an alkylating agent. As a dialkylating agent, BCNU is able to form interstrand crosslinks in DNA, which prevents DNA replication and DNA transcription. Carmustine having an empirical formula of C₅H₉C₁₂N₃O₂ and a molecular weight of 214.06 g/mol. Carmustine is the international commonly accepted name for 1,3-bis-(2-chloroethyl)-1-nitrosourea and has the structure of formula (I)

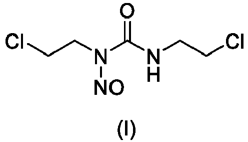

BiCNU^{®} (carmustine for injection) is one of the nitrosoureas used in the treatment of certain neoplastic diseases.

Processes for preparing urea compounds are known from the prior art. Franca Bigi et al. (Selected syntheses of ureas through phosgene substitutes, Green Chemistry, vol. 2, no. 4, (2000), pages 140-148, XP055087859) generically discloses processes for the synthesis of substituted ureas, wherein said processes involve essentially two-steps; i.e. meaning that isolation of the carbonylimidazolide intermediate is carried out, which is further subjected to conversion to the corresponding urea product.

Takeda Kazuyoshi et al. (Studies on activating methods of functional groups. Part X. Convenient methods for syntheses of active carbamates, ureas and nitrosoureas using N,N'disuccinimido carbonate (DSC), Tetrahedron Letters, vol. 24, no. 42, (1983), pages 4569-4572, XP009098593) discloses a method for the synthesis of ureas and nitrosoureas employing N,N'-disuccinimido carbonate reagent (DSC).

Angelica Ion et al. (Synthesis of symmetrical or asymmetrical urea compounds from CO2 via base catalysis, Green Chemistry, vol. 9, (2006), pages 159-161, DOI: 10.1039/b612403h) discloses a direct carbonylation process of amines by carbon dioxide as a carbonylating moiety.

J.P Kamlesh et al. (Unprecedented "In Water" Imidazole Carbonylation: Paradigm Shift for Preparation of Urea and Carbamate, Organic Letters. vol. 14, no. 11, (2012), XP055129354) discloses a process for the preparation of unsymmetrical urea compounds, carbamates and thiocarbamates trough reaction employing water as solvent.

Process for preparation of carmustine and its urea intermediate of formula (II) is known from various prior arts such as US6096923, FR2589860, RO56999A2 and US4128639.

US4028410 discloses a process of producing carmustine by nitrosation of 1,3-bis(2-chloroethyl)-urea employing as a nitrosating agent dinitrogen trioxide (N₂O₃) in molar excess.

The reported processes to prepare carmustine can be summarized as following scheme:

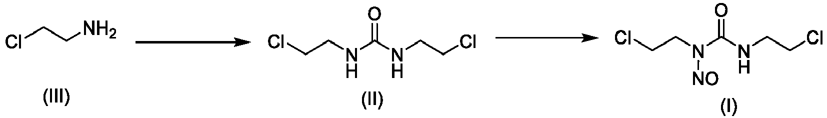

All the reported processes use phosgene for preparation of intermediate of formula (II).

Phosgene is highly toxic substance that exists as a gas at room temperature. According to the National Institute for Occupations Safety and Health (NIOSH), a toxic level of phosgene that can place a person's life and well-being in jeopardy can be as low as 2 parts per million.

In view of the hazardous nature of phosgene, it is highly desirable to replace phosgene in the process for preparation of carmustine.

The present invention provides a process to prepare carmustine wherein the use of hazardous reagents specifically phosgene is completely avoided. Hence the process of present invention is safe, simple and industrial friendly.

### OBJECTS OF THE INVENTION

The main object of the present invention is to provide a process for preparation of carmustine of formula (I)

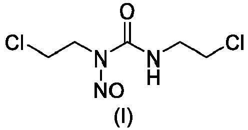

Object of present invention is to provide a process for preparation of comprising a step of reacting 2-chloroethylamine with 1,1'-carbonyldiimidazole in the presence of a base to obtain 1,3-bis[2-hydroxyethyl]urea (II).

Yet another object of present invention is to provide a process for preparation of carmustine comprising steps of:
a) reacting 2-chloroethylamine with 1,1'-carbonyldiimidazole in the presence of a base;
b) converting 1,3-bis(2-hydroxyethyl)urea (II) to carmustine (I).

Yet another object of present invention is to provide a process for preparation of carmustine comprising steps of:
a) reacting 2-chloroethylamine with 1,1'-carbonyldiimidazole in the presence of a base;
b) subjecting 1,3-bis(2-hydroxyethyl)urea (II) obtained in step a); to nitrosation; and
c) isolating carmustine (I).

Yet another object of present invention is to provide a process for preparation of carmustine comprising steps of:
a) reacting 2-chloroethylamine with 1,1'-carbonyldiimidazole in the presence of a base;
b) reacting 1,3-bis(2-hydroxyethyl)urea (II) obtained in step a) with sulphuric acid and sodium nitrite; and
c) isolating carmustine (I).

### SUMMARY OF THE INVENTION

The main aspect of the present invention is to provides a process for preparation of carmustine (I)

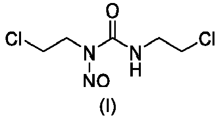

In an aspect, the present invention provides a process to prepare carmustine (I) wherein the process comprises a step of reacting 2-chloroethyl amine (III) or its salt with 1,1'-carbonyldiimidazole to obtain 1,3-bis(2-chloroethyl)urea (II).

In another aspect, the present invention provides a process to prepare carmustine wherein the process comprises:
a) reacting 2-chloroethyl amine or its salt with a reagent selected from 1,1'-carbonyldiimidazole in the presence of a base to obtain 1,3-bis (2-chloroethyl)urea (II);
b) reacting 1,3-bis(2-chloroethyl)urea with nitrosating reagent to obtain carmustine.

In another aspect, the present invention provides a process to prepare carmustine wherein the process comprises steps of:
a) reacting 2-chloroethyl amine or its salt with a reagent selected from 1,1'-carbonyldiimidazole in the presence of a base to obtain 1,3-bis(2-chloroethyl)urea (II);
b) reacting 1,3-bis(2-chloroethyl)urea in presence of sulphuric acid and sodium nitrite; and
c) isolating carmustine (I).

### DETAILED DESCRIPTION OF THE INVENTION

The main embodiment of the present invention is to provide a process for preparation of carmustine (I).

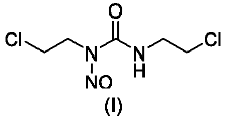

In another embodiment, the present invention provides a process wherein 2-chloroethylamine or its salts are converted to 1,3-bis(2-chloroethyl)urea (II) in absence of phosgene. The process of the present invention is carried out in the presence of 1,1'-carbonyldiimidazole.

Processes known in the art generally uses phosgene for above conversion. Phosgene is very hazardous chemical and need to be avoided on large scale preparation. Present invention provides a process for preparation of carmustine wherein the step of preparing intermediate (II) entirely avoids use of phosgene, and thus make the overall process simple yet efficient and environment friendly.

In another embodiment the present invention provides a process to prepare carmustine as depicted by following scheme:

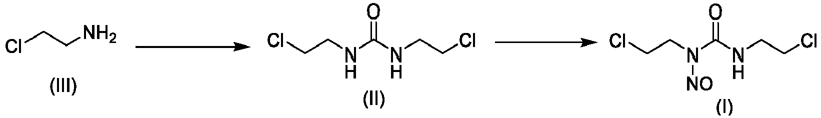

Conversion of 2-chloroethyl amine of formula (III) to an intermediate of formula (II) can be carried out in presence of a reagent selected from 1,1'-carbonyldiimidazole.

This reaction can be carried out in presence of base and solvent. Base for the purpose of above conversion can be selected from any suitable organic or inorganic base. Preferably the reaction to prepare urea intermediate is carried out in presence of triethylamine.

Solvent can be selected from any organic solvent or mixture of solvents suitable for the reaction. This reaction is generally carried out at temperature in the range from 5°-50°C.

In next step of the process intermediate of formula (II) is subjected to nitrosation.

Carmustine is nitroso-urea compound so the synthesis involves first step of obtaining urea intermediate i.e. intermediate of formula (II) and then converting urea intermediate to carmustine by nitrosation.

The nitrosation reaction can be carried out in presence of sulphuric acid and sodium nitrite. The reaction is carried out in presence of solvent. The reaction can be carried out in presence of single solvent, mixture of solvents or in two phase solvent system. Preferably the nitrosation reaction is carried out in presence of dichloromethane and water. Nitrosation is generally carried out at a temperature in range of -5°C to 10°C.

The nitrosation reaction can be carried out by using suitable nitrosating agent selected from dinitrogen trioxide, sodium nitrite and aq. HCl, sodium nitrite and H₂SO4, sodium nitrite and HCOOH and sodium nitrite and CH₃COOH.

The final product i.e. carmustine can be isolated by removal of solvent from reaction mixture or separating the layers and isolating the product from organic layer.

In an embodiment the product can be isolated by removing the solvent from the reaction mixture, followed by addition of suitable solvent to the residue to isolate the product.

In another embodiment carmustine can be isolated by removing solvent from organic layer, adding suitable solvent to the residue to dissolve carmustine, followed by addition of anti-solvent to isolate carmustine. Solvents used to dissolve residue (i.e. carmustine) can be selected form group consisting of ether such as diethyl ether, methyl tert-butyl ether, alkyl acetate such as ethyl acetate, aromatic hydrocarbon such as toluene and alkanol such as ethanol, isopropanol. Anti-solvent used to isolate carmustine can be selected from group consisting of alkanes such as heptane, hexane, or pentane, cycloalkanes such as cyclohexane and water.

In general the solvents used for the purpose of present invention i.e. used for conducting the reaction or isolating the product can be selected form any suitable solvent such as alkanol, ketone, ether, hydrocarbon, chlorinated solvents, water, aprotic solvents, alkanes or mixture thereof.

The process of the present invention uses non-hazardous and easily available reagents for preparation of carmustine, thus, makes the process commercially viable.

### EXAMPLES:

### Example 1: Preparation of 1,3-(bis(2-chloroethyl)urea (II)

2-chloroethyl amine hydrochloride (20g) and triethylamine (90ml) were added to a round bottom flask. The resultant reaction mixture was cooled to 10-20°C, to this 15 g of 1,1'-carbonyldiimidazole (CDI) was added, the reaction mixture was stirred for 2 hrs at 35-40°C. After completion of reaction the reaction mixture was cooled to 20-25°C, solvent was distilled of to obtain residue. 100 ml water was added to the residue and the mixture was filtered, the solid thus obtained was dried to give 13g of title compound.

### Example 2: Preparation of carmustine (I)

Water (80ml) was taken in a round bottom flask, cooled to 0-5°C, to this sulphuric acid (38g) was added. This was followed by addition of 80 ml of methylene dichloride and 10g of 1,3-bis(2-chloroethyl)urea (II). The reaction mixture was stirred while maintaining the temperature at about 0-5°C, to this sodium nitrite solution (24g sodium nitrite in 120ml water) was added. The reaction mixture was stirred at the same temperature for 2 hrs, after completion of reaction, layers separated, organic layer was washed with sodium sulfate, and the solvent was distilled off. To the residue a mixture of n-heptane and methyl tertiary butyl ether was added. Reaction mixture was stirred for 1 hr, filtered and the solid thus obtained was dried to give 12g of title compound.

## Claims

1. A process for preparation of carmustine (I) 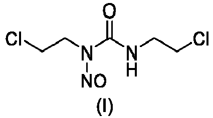 comprising steps of:
a) converting 2-chloroethylamine (III) or a salt thereof to 1,3-bis(2-chloroethyl)urea (II) 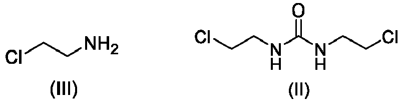 with 1,1-carbonyldiimidazole in the presence of a base; and
b) converting urea of formula (II) to carmustine (I) with nitrosating reagent in the presence of solvent.

2. The process according to claim 1, wherein nitrosating reagent is selected from group consisting of di-nitrogen trioxide, sodium nitrite and acid.

3. The process according to claim 2, wherein acid is selected from hydrochloric acid, sulphuric acid, formic acid and acetic acid.

4. The process according to any of the preceding claims, wherein nitrosating reagent is sodium nitrite and acid is sulphuric acid.

5. The process according to claim 1, wherein base used in step (a) is triethylamine.

6. The process according to claim 1, wherein solvent used in nitrosation is dichloromethane and water.

## Patentansprüche

1. Verfahren zur Herstellung von Carmustin (I) 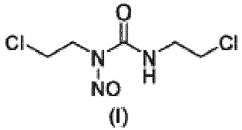 das folgende Schritte umfasst:
a) Umwandeln von 2-Chlorethylamin (III) oder einem Salz davon in 1,3-Bis(2-chlorethyl) harnstoff (II) 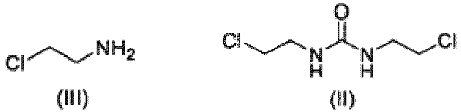 mit 1,1-Carbonyldiimidazol in Gegenwart einer Base und
b) Umwandeln des Harnstoffs der Formel (II) in Carmustin (I) mit einem Nitrosierungsreagenz in Gegenwart von Lösungsmittel.

2. Verfahren nach Anspruch 1, wobei das Nitrosierungsreagenz aus der Gruppe bestehend aus Distickstofftrioxid, Natriumnitrit und Säure ausgewählt wird.

3. Verfahren nach Anspruch 2, wobei die Säure aus Salzsäure, Schwefelsäure, Ameisensäure und Essigsäure ausgewählt wird.

4. Verfahren nach einem der vorhergehenden Ansprüche, wobei es sich bei dem Nitrosierungsreagenz um Natriumnitrit handelt und es sich bei der Säure um Schwefelsäure handelt.

5. Verfahren nach Anspruch 1, wobei es sich bei der in Schritt (a) verwendeten Base um Triethylamin handelt.

6. Verfahren nach Anspruch 1, wobei es sich bei dem bei der Nitrosierung verwendeten Lösungsmittel um Dichlormethan und Wasser handelt.

## Revendications

1. Procédé pour la préparation de carmustine (I) 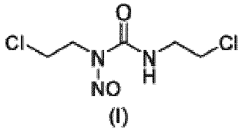 comprenant les étapes de:
a) conversion de la 2-chloroéthylamine (III) ou d'un sel correspondant en 1,3-bis(2-chloroéthyle)urée (II) 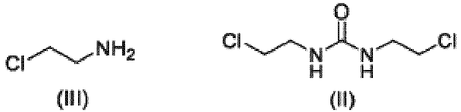 avec du 1,1-carbonyldiimidazole en la présence d'une base; et
b) conversion de l'urée de formule (II) en carmustine (I) avec un réactif de nitrosation en la présence de solvant.

2. Procédé selon la revendication 1, le réactif de nitrosation étant choisi dans le groupe constitué par le trioxyde de diazote, le nitrite de sodium et un acide.

3. Procédé selon la revendication 2, l'acide étant choisi parmi l'acide chlorhydrique, l'acide sulfurique, l'acide formique et l'acide acétique.

4. Procédé selon l'une quelconque des revendications précédentes, le réactif de nitrosation étant le nitrite de sodium et l'acide étant l'acide sulfurique.

5. Procédé selon la revendication 1, la base utilisée dans l'étape (a) étant la triéthylamine.

6. Procédé selon la revendication 1, le solvant utilisé dans la nitrosation étant le dichlorométhane et l'eau.
